# EUROPÄISCHE PATENTSCHRIFT

(11) **EP 2 691 015 B1**
(45) Veröffentlichungstag und Bekanntmachung des Hinweises auf die Patenterteilung: **18.07.2018**
(21) Anmeldenummer: 12725297.1
(22) Anmeldetag: 30.03.2012
(51) Int. Cl.: A61B 5/00, A61N 1/372

(54) **ÜBERWACHUNGSSYSTEM**
MONITORING SYSTEM
SYSTÈME DE SURVEILLANCE

(30) Priorität: 30.03.2011 DE 102011001678
(43) Veröffentlichungstag der Anmeldung: 05.02.2014
(73) Patentinhaber: medic assist GmbH & Co. KG, 44789 Bochum (DE)
(72) Erfinder: MASSMANN, Clemens, 76131 Karlsruhe (DE)
(74) Vertreter: Limbeck, Achim
(86) Internationale Anmeldenummer: PCT/DE2012/100087
(87) Internationale Veröffentlichungsnummer: WO 2012/130232

(56) Entgegenhaltungen:
- US-A1- 2005 038 482
- US-A1- 2008 172 109
- US-A1- 2010 179 618

## Beschreibung

Die Erfindung betrifft ein Überwachungssystem zur Überwachung eines Drittgerätes, insbesondere eines medizinischen Gerätes, umfassend eine Überwachungsvorrichtung (2) und das zu überwachende Drittgerät, wobei das Drittgerät eine Signalvorrichtung zur Anzeige des Gerätezustands und/oder relevanter Geräteeigenschaften aufweist und die Überwachungsvorrichtung dem Drittgerät zugeordnet ist, ohne dass hiermit ein Eingriff in das Gehäuse des Drittgerätes und/oder die Funktionalität des Drittgerätes verbunden ist.

In diesem Zusammenhang ist aus der US 2010/179618 A1 eine implantierbare medizinische Vorrichtung vorbekannt, die in der Lage ist, Fehlermeldungen über eine Ladespule zu kommunizieren, insbesondere, wenn die herkömmlichen Telemetrie-Nationalitäten ausgefallen sind. Ein typisches Anwendungsbeispiel für die vorbekannte Lösung ist ein implantierbarer Herzschrittmacher, dem ein externer Controller zur Überwachung der Funktionalität des Herzschrittmachers zugeordnet ist. Hierzu können Daten durch die miteinander gekoppelten Spulen des Controllers und des Herzschrittmachers ausgetauscht werden für den Fall, dass es sich dabei um Fehlermeldungen handelt, werden diese zunächst an einen in den Herzschrittmacher integrierten Microcontroller übermittelt und dann über eine Modulationseinheit über die erwähnte Spule induktiv nach entsprechender Modulation an den induktiv angeschlossenen externen Controller übertragen.

Ein weiteres Überwachungssystem zum Erfassen und Übertragen von Statusinformationen einer tragbaren medizinischen Vorrichtung ist aus dem europäischen Patent EP 1435076 B1 vorbekannt.

Obwohl medizinische Vorrichtungen zur Überwachung lebenserhaltender Funktionen, auf die im Notfall zugegriffen wird, üblicherweise mit einer Anzeige versehen sind, die die Funktionsfähigkeit des entsprechenden Gerätes signalisiert, sind diese Anzeigen in der Regel im täglichen Gebrauch nicht ausreichend. Wenn beispielsweise eine tragbare medizinische Vorrichtung, wie etwa ein Defibrillator, in einem Schrank, Kasten o. ä. aufbewahrt wird, beispielsweise um den unbefugten Zugriff zu verhindern, so wird eine optische oder akustische Zustandsanzeige in der Regel unbeachtet bleiben.

Ausgehend von diesem Stand der Technik soll gemäß der vorbekannten Lösung ein Gerät entwickelt werden, mit dem die angezeigten Statusinformationen erfasst und gegebenenfalls an eine entfernte Überwachungsvorrichtung übermittelt werden können. Dabei ist in der Regel bei derartigen Zusatzeinrichtungen zu beachten, dass ein Eingriff in das bereits bestehende, zu überwachende Gerät ohne Verlust der medizinischen Zulassungen in diesem Zusammenhang nicht möglich ist und daher möglichst vermieden werden soll. Die vorbekannte Lösung schlägt daher vor, dem medizinischen Gerät eine Erfassungsvorrichtung zum Detektieren einer Zustandsänderung des Statusanzeigers der tragbaren medizinischen Vorrichtung zuzuordnen, wobei der Empfänger zum Detektieren der Zustandsänderung mit einer Übertragungseinrichtung gekoppelt ist, die eine detektierte Zustandsänderung der Statusanzeige an einen entfernten Empfänger ermöglicht.

Bei richtigem Verständnis handelt es sich bei der gemäß dem europäischen Patent überwachten Zustandsanzeige um eine optisehe Anzeige, die mit einer optischen Erfassungsvorrichtung überwacht wird. Dies setzt zunächst voraus, dass zwischen dem Empfänger der Überwachungsvorrichtung und der Zustandsanzeige ein ungehinderter optischer Kanal zur Verfügung steht. Je nach Lage und Aufbewahrung der zu überwachenden medizinischen Vorrichtung ist es allerdings in vielen Fällen schwierig, der überwachten medizinischen Vorrichtung ein Zusatzgerät zuzuordnen, das ungehinderten Blickkontakt mit der Zustandsanzeige des überwachten Gerätes hat. Doch selbst wenn dies gewährleistet ist, kann der optische Kanal aus vielen Gründen gestört sein - etwa durch Streu- oder Blendlicht -, wobei in diesem Falle ein Notsignal detektiert werden müsste, obgleich sich an dem Zustand des überwachten Gerätes nichts geändert hat. Im Übrigen führt bei dem aus dem europäischen Patent bekannten System eine Funktionsstörung der Zustandsanzeige ebenfalls zu einem Notsignal, auch dann, wenn die Funktion des medizinischen Gerätes ansonsten intakt ist. Für den Fall, dass bei der vorbekannten Überwachungseinrichtung die Zustandsanzeige so konfiguriert ist, dass sie lediglich eine Betriebsstörung aktiv anzeigt, kann die vorbekannte Überwachungseinrichtung folglich bei defekter Zustandsanzeige nicht mehr funktionieren.

Ausgehend von diesem Stand der Technik liegt der Erfindung die Aufgabe zu Grunde, eine Überwachungsvorrichtung für ein Drittgerät, insbesondere aber für eine medizinische Vorrichtung, zu schaffen, deren Funktion unabhängig von der Funktion einer etwaigen Zustandsanzeige des Gerätes ist und die im Übrigen auch dann funktioniert, wenn der optische Kanal zwischen einer Erfassungsvorrichtung und dem überwachten Gerät gestört ist.

Die erfindungsgemäße Aufgabe wird durch ein System mit den Merkmalen des Hauptanspruchs gelöst. Vorteilhafte Ausgestaltungen der Erfindung können den abhängigen Ansprüchen 2 bis 8 entnommen werden.

Dadurch, dass im Rahmen der erfindungsgemäßen Lösung nicht eine Zustandsanzeige überwacht wird, sondern vielmehr ein im Gerät zur Aufrechterhaltung der gerätetechnischen Funktionen notwendiger Strom, hängt die Überwachung nicht von der Funktion einer möglichen Zustandsanzeige des Gerätes ab. Anders ausgedrückt: es wird nicht eine Zustandsanzeige, sondern vielmehr die Funktion des Gerätes selbst überwacht. Dies gelingt, indem der relevante Strom mittels einer Spule durch Ausnutzung der Induktionswirkung des Stromes kontaktlos überwacht wird. Hierzu ist die Spule in der Nähe der Spannungsversorgung des überwachten Gerätes angeordnet. Nachdem die Überwachungsvorrichtung mittels induktiver Kopplung arbeitet, ist die Funktionsfähigkeit des Überwachungssystems unabhängig von einem direkten oder indirekten Sichtkontakt mit einer Zustandsanzeige.

Ein weiterer Vorteil der induktiven Überwachung des Gerätezustandes besteht darin, dass auch bei installierter Überwachungsvorrichtung keinerlei Veränderung oder Beeinträchtigung des User-Interfaces des überwachten Gerätes erforderlich ist. Im übrigen leistet die Überwachung neben der Überwachung des Gerätezustandes ggf. auch eine Überwachung auf mögliche Änderungen des Gerätezustandes.

In vorteilhafter Ausgestaltung ist die Spule zur Detektion eines signifikanten Stromes in der Nähe der Batterie des Drittgerätes angeordnet. Dieser Anordnung liegt die Erkenntnis zu Grunde, dass die zu überwachenden Drittgeräte, insbesondere medizinische Geräte, zwar ständig funktionsfähig und betriebsbereit sein müssen, aber oftmals nicht ständig in Betrieb stehen, sondern vielmehr in einer Bereitstellung. Dieser Bereitschaftszustand wird in der Regel als so genannter Sleep-Modus bzw. Stromsparmodus bezeichnet, in dem lediglich die Funktionen des Gerätes aufrechterhalten sind, die notwendig sind, um die Betriebsbereitschaft des Gerätes für den Ernstfall zu erhalten. Die Funktionsfähigkeit des Gerätes ist also dann gegeben, wenn zumindest noch der zur Aufrechterhaltung des Sleep-Modus notwendige Strom von dem Gerät bezogen wird. Dieser Strom wird entweder von einer geräteexternen oder einer geräteinternen Spannungsversorgung geliefert, also beispielsweise im Falle einer Insellösung und netzunabhängigen Geräteaufstellung von einer in das Drittgerät integrierten Batterie. Um den hier fließenden Strom detektieren zu können, ist die Spule in der Nähe der Spannungsversorgung bzw. der Batterie des Drittgerätes oder in der Nähe sonstig für die Zwecke der Überwachung signifikanter Leiterbahnen angeordnet.

Bei einer solchen Anordnung hat es sich bewährt, wenn dann mit der entsprechend angeordneten Spule auch der zur Aufrechterhaltung der in einem Sleep-Modus aktiven Funktionen benötigte Stromfluss mit der Spule detektiert wird.

In vorteilhafter Weiterbildung muss die mit dem Überwachungssystem realisierte Überwachung des Drittgerätes nicht auf eine passiv reagierende Überwachungseinheit beschränkt bleiben, sondern kann mittels eines in die Überwachungseinheit integrierten Aktors eine Gerätezustandsanzeige aktiv abrufen, die dann wieder eine mit der Anzeige und dem Gerätezustand korrelierenden Stromfluss bzw eine korrelierende Änderung des Stromflusses des mit der Spule überwachten Stroms bewirkt, die dann mittels der Spule detektiert wird. Auf diese Weise ist eine aktive und damit präventive Überwachung des Drittgerätes realisiert.

Auf Grund der induktiven Wirkung des Stromflusses erzeugt die Spule einen Wechselstrom bzw. ein periodisches Signal oder ein Impuls-Signal, das dem detektierten Strom proportional ist. Dieses Signal kann dann an eine gegebenenfalls entfernte Auswerteeinheit übermittelt werden oder von einer solchen Auswerteeinheit abgefragt werden.

In vorteilhafter Weiterbildung der erfindungsgemäßen Lösung kann hierzu die Erfassungseinheit, in der die Spule integriert ist, mit einem Funkübertragungs-Modul zur drahtlosen Übertragung und/oder Fernabfrage des mittels der Spule detektierten Signals versehen sein.

In besonders vorteilhafter Verwendung wird das Überwachungssystem in Verbindung mit einem Defibrillator eingesetzt, der insbesondere in öffentlichen Einrichtungen für den Notfall vorgehalten wird. Mittels des erfindungsgemäßen Überwachungssystems ist sichergestellt, dass der Defibrillator im Ernstfall auch funktionsfähig ist. Ein Strom-Überwachungsgerät mit induktivem Empfänger geeignet für ein nicht implantierbares medizinisches Drittgerät ist aus WO9941616 bekannt. Die Erfindung wird nachstehend anhand eines in der Zeichnung nur schematisch dargestellten Ausführungsbeispiels näher erläutert:

Es zeigt:

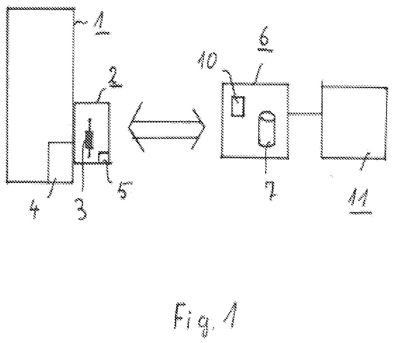

- Figur 1: ein Überwachungssystem für einen Defibrillator in einem Blockschaltbild.

Das Blockschaltbild zeigt zunächst ein Überwachungssystem umfassend ein zu überwachendes Drittgerät, hier einen Defibrillator 1, dem eine Überwachungsvorrichtung 2 mit integrierter Spule 3 zugeordnet ist. Der Defibrillator 1 wird üblicherweise in einem öffentlich zugänglichen Gebäude für den Notfall vorgehalten und ist zur Aufrechterhaltung der Funktionsfähigkeit dabei von der übrigen Stromversorgung unabhängig, d. h. mit einer Batterie 4 zur Aufrechterhaltung des Betriebszustandes versehen. Die Überwachungsvorrichtung 2 mit der integrierten Spule 3 ist dabei in der Nähe der Batterie 4 angeordnet, so dass der im Sleep-Modus des Defibrillators 1 fließende Strom zur Aufrechterhaltung der grundsätzlichen Betriebsfähigkeit des Defibrillators 1 mittels der Spule 3 im Wege der induktiven Kopplung detektiert werden kann.

Auf Grund der induzierten Wechselspannung erzeugt die Spule 3 ein periodisches Signal, das zu dem überwachten Stromfluss innerhalb des Defibrillators 1 proportional ist. Dieses Signal oder ein zu dem Signal proportionales Signal, insbesondere ein mittels eines Messverstärkers verstärktes Signal, kann dann mittels eines ebenfalls in die Überwachungsvorrichtung 2 integriertes Funkübertragungsmoduls 5 drahtlos an eine im Regelfall entfernte Auswerteeinheit 6 übermittelt werden. Die Auswerteeinheit 6 enthält üblicherweise eine Prozessoreinheit 7 und ein Speicherelement 10 zur Speicherung der von der Überwachungsvorrichtung 2 übermittelten Signale. Für den Fall, dass die von Überwachungsvorrichtung 2 an die Auswerteeinheit 6 übermittelten Signale vorgegebene Schwellwerte über- oder unterschreiten, wird von der Auswerteeinheit 6 ein Alarmsignal detektiert. Das Alarmsignal wird dabei üblicherweise drahtlos oder drahtgebunden direkt in eine zentrale Datenverarbeitungseinheit 11, etwa einer Notrufzentrale oder einer Servicezentrale, eingespeist. Die Servicezentrale wird dann einen Techniker beauftragen, der das überwachte Drittgerät entweder austauscht oder repariert, wobei dies nicht Gegenstand der hier zu diskutierenden Erfindung ist.

Vorstehend ist somit ein Überwachungssystem, insbesondere zur Überwachung medizinischer Notfallgeräte, beschrieben, das es erlaubt, unabhängig von der Zustandsanzeige des überwachten medizinischen Gerätes selbst den Gerätezustand, insbesondere dessen Betriebsbereitschaft, zu erfassen und gegebenenfalls an eine entfernte Auswerteeinheit zu übermitteln, so dass von einer Zentraleinheit aus die dezentral verteilt angeordneten medizinischen Vorrichtungen, insbesondere Defibrillatoren, auf ihre Funktionsfähigkeit hin überwacht und gegebenenfalls repariert, gewartet oder ausgetauscht werden können.

## Patentansprüche

1. Überwachungssystem zur Überwachung eines Drittgerätes, insbesondere eines medizinischen Gerätes, umfassend eine Überwachungsvorrichtung (2) und das zu überwachende Drittgerät, wobei das Drittgerät eine Signalvorrichtung zur Anzeige des Gerätezustands und/oder relevanter Geräteeigenschaften aufweist und die Überwachungsvorrichtung (2) dem Drittgerät zugeordnet ist, ohne dass hiermit ein Eingriff in das Gehäuse des Drittgerätes und/oder die Funktionalität des Drittgerätes verbunden ist, wobei, das Drittgerät ein nichtimplantierbares medizinisches Gerät, vorzugsweise ein nicht-implantierbarer Defibrillator (1) ist und die Überwachungsvorrichtung (2) einen induktiven Empfänger, vorzugsweise eine Spule (3), umfasst, die dem Gehäuse des Drittgerätes derart zugeordnet ist, dass mittels des Empfängers ein für den Gerätezustand und/oder eine relevante Geräteeigenschaft des Drittgerätes signifikanter Strom und/oder Stromänderung überwacht wird, wobei besagter Empfänger (3) in der Nähe der Spannungsversorgung des überwachten Drittgerätes ist.

2. Überwachungssystem nach Anspruch 1, wobei der Empfänger eine Spule umfasst, **dadurch gekennzeichnet, dass** die Spule (3) in der Nähe der Batterie (4) des Drittgerätes, außerhalb des Gehäuses des überwachten Drittgerätes angeordnet ist.

3. Überwachungssystem nach Anspruch 1 wobei der Empfänger eine Spule umfasst, oder Anpruch 2, **dadurch gekennzeichnet, dass** mittels der Spule (3) ein zur Aufrechterhaltung der in einem Sleep-Modus aktiven Funktionen, insbesondere der Anzeige der ordnungsgemäßen Gerätefunktion, des Drittgerätes benötigter Stromfluss innerhalb des Drittgerätes detektiert wird.

4. Überwachungssystem nach Anspruch 1, wobei der Empfänger eine Spule umfasst, oder Ansprüche 2-3, **dadurch gekennzeichnet, dass** das Drittgerät mittels der Signalvorrichtung aktiv überwacht ist, indem die Überwachungsvorrichtung derart mit dem Drittgerät gekoppelt ist, dass die Überwachungsvorrichtung mittels eines Aktors eine Zustandsanzeige der Signalvorrichtung auslösbar ist, die anschließend mittels der Spule (3) der dem Drittgerät zugeordneten Empfängereinheit der Überwachungsvorrichtung erfassbar und anschließend mittels eines Speicherelementes (10) dieser Empfängereinheit speicherbar und/oder an eine gegebenenfalls entfernt angeordnete Auswertevorrichtung übermittelbar ist.

5. Überwachungssystem nach Anspruch 1, wobei der Empfänger eine Spule umfasst, oder Ansprüche 2-4, **dadurch gekennzeichnet, dass** mittels der Spule (3) der Signalstrom für eine Sprachausgabe des überwachten Drittgerätes und/oder der Signalstrom für einen Selbsttest des überwachten Drittgerätes überwacht wird.

6. Überwachungssystem nach Anspruch 1, wobei der Empfänger eine Spule umfasst, oder Ansprüche 2-5, **dadurch gekennzeichnet, dass** die Spule (3) in Abhängigkeit von dem detektierten Strom ein mit dem Gerätezustand und/oder einer relevanten Geräteeigenschaft des überwachten Drittgerätes korrelierendes periodisches Signal detektiert, das einer, gegebenenfalls entfernten, Auswerteeinheit (6) übermittelt wird und/oder fernabfragbar ist.

7. Überwachungssystem nach Anspruch 6, **dadurch gekennzeichnet, dass** die Spule (3) in eine Erfassungseinheit integriert ist, die Funkübertragungs-Modul (5) zur Übertragung und/oder Fernabfrage des mittels der Spule (3) detektierten Signals aufweist.

8. Verwendung des Überwachungssystems nach Anspruch 1, wobei der Empfänger eine Spule umfasst, oder Ansprüche 2-7, zur Überwachung eines nichtimplantierbaren Defibrillators (1).

## Claims

1. Monitoring system for monitoring a third party device, in particular a medical device, comprising a monitoring device (2) and the third party device to be monitored, whereas the third party device comprises a signalling device to communicate the status of the third party device and/or other relevant third party device properties, and whereas the monitoring device (2) is associated to the third party device without any mechanical intervention into the housing of the third party device or its function, and whereas the third party device is a non-implantable medical device, preferably a non-implantable defibrillator (1), and the monitoring device (2) comprises an inductive receiver, preferably a coil (3) that is arranged near the housing of the third party device in such a way that the monitoring system is able to monitor the state of the third party device by receiving information on the third party device status and/or relevant device properties by monitoring the significant electrical current and/or changes of the electrical current in the third party device, whereby the described receiver (3) is arranged near the electrical power supply of the third party device to be monitored.

2. Monitoring system according to Claim 1,
whereby the receiver comprises a coil **characterized in that** the coil (3) is located near the battery (4) of the third party device and outside the housing of the third party device to be monitored.

3. Monitoring system according to Claim 1,
whereby the receiver comprises a coil,
or according to Claim 2,
**characterized in that** by means of the coil (3) an electrical current within the third party device required for sustaining third party device functions active during a sleep mode, especially for the display of the proper device functions, is detected.

4. Monitoring system according to Claim 1,
whereby the receiver comprises a coil,
or according to Claims 2 and 3,
**characterized in that** the third party device is actively monitored by means of a signalling device in a manner that the monitoring device is coupled to the third party device in such a way that the monitoring device is triggered by means of an actuating status signal, which is detected by the coil (3) connected to the monitoring device described here, and which can be recorded by a storage device (10) associated to the receiver device of the monitoring system and/or a possibly remote storage and evaluation system associated with this system.

5. Monitoring system according to Claim 1,
whereby the receiver comprises a coil,
or according to Claims 2 through 4,
**characterized in that** the signal current for a speech output of the monitored third party device and/or or the signal current for a self-test of the monitored third party device is monitored.

6. Monitoring system according to Claim 1,
whereby the receiver comprises a coil,
or according to Claims 2 through 5,
**characterized in that** the coil (3) detects a signal depending on the detected correlating periodic signal generated by the monitored third party device depending on the status and/or device properties of the monitored device that can be remotely transmitted or transferred or requested by an, also remote, evaluation device (6).

7. Monitoring system according to Claim 6,
**characterized in that** the coil (3) is integrated into a recording device comprising a remote wireless transmission module (5) for the remote transmission or request of the signals detected by the coil (3).

8. Use of the monitoring system according to Claim 1,
whereby the receiver comprises a coil,
or according to Claims 2 through 7,
for monitoring a non-implantable defibrillator (1).

## Revendications

1. Système de suivi pour la surveillance d'un troisième dispositif, il s'agit notamment d'un dispositif médical comprenant un dispositif de surveillance (2) et le périphérique tiers à surveiller, tout en notant que le périphérique tiers comporte un dispositif de signalisation pouvant indiquer l'état de l'appareil et/ou les caractéristiques du périphérique concerné et le dispositif de surveillance (2) est associé au troisième périphérique sans aucune intervention dans le boîtier de la troisième unité et/ou sans que la fonctionnalité du troisième appareil soit connectée, et dans la mesure où, le troisième dispositif est un appareil médical non implantable, voire que ce soit de préférence, un défibrillateur non implantable (1) et dans la mesure où le dispositif de surveillance (2) comprend un récepteur inductif, voire que ce soit de préférence,une bobine (3), qui est alors associé au boîtier du troisième dispositif de sorte qu'au moyen du récepteur, un flux de courant significatif et/ou le changement de courant relativement à l'état du dispositif et/ou une propriété pertinente du troisième dispositif soient surveillés à condition que ledit récepteur (3) soit proche de l'alimentation électrique du dispositif tiers surveillé.

2. Système de surveillance selon la revendication 1, où le récepteur est composé d'une bobine, **caractérisée par le fait que** la bobine (3) soit disposée à proximité de la batterie (4) de la troisième unité, à l'extérieur du boîtier de l'appareil tiers surveillé.

3. Système de surveillance selon la revendication 1, où le récepteur est composé d'une bobine, ou selon la revendication 2, **caractérisé par le fait qu'**au moyen de la bobine (3), un flux de courant requis pour maintenir les fonctions actives en mode veille, en particulier l'indication de la fonction de dispositif appropriée, du dispositif tiers est détecté au sein du dispositif tiers.

4. Système de surveillance selon la revendication 1, où le récepteur comprend une bobine, ou selon les revendications 2 et 3, **caractérisé en ce que** le dispositif de tiers est activement surveillé au moyen du dispositif de signalisation de l'équipement de surveillance qui est ainsi couplé au troisième dispositif, de sorte que le dispositif de surveillance est déclenchable au moyen d'un actionneur avec affichage de l'état de l'appareil de signal, qui ensuite est détectable par l'intermédiaire de la bobine (3) de l'unité réceptrice de surveillance associée au périphérique tiers et ensuite au moyen d'un élément de stockage (10), cet appareil récepteur peut être mémorisé et/ou transmis à un dispositif d'évaluation éventuellement situé à distance.

5. Système de surveillance selon la revendication 1, où le récepteur comprend une bobine, ou selon les revendications 2-4, **caractérisées en ce que** par l'intermédiaire de la bobine (3), le signal actuel pour une sortie vocale du troisième dispositif surveillé et/ou le signal de puissance pour le contrôle automatique du périphérique tiers, est surveillé.

6. Système de surveillance selon la revendication 1, où le récepteur comprend une bobine, ou selon les revendications 2 à 5, **caractérisées en ce que** la bobine (3) repère, en fonction du courant détecté, un signal périodique corrélant avec l'état du dispositif et/ou un dispositif pertinent caractéristique du troisième dispositif surveillé qui est transmis à une unité d'évaluation (6) éventuellement distante et/ou interrogeable à distance.

7. Système de surveillance selon la revendication 6, **caractérisé en ce que** la bobine (3) est intégrée dans une unité de détection qui comporte un module de transmission radio (5) pour l'émission et/ou l'interrogation à distance du signal détecté au moyen de la bobine (3).

8. Utilisation du système de surveillance selon la revendication 1, où le récepteur comprend une bobine, ou selon les revendications 2 à 7, pour surveiller un défibrillateur non implantable (1)
